# EUROPEAN PATENT APPLICATION

(11) **EP 4 205 651 A1**
(43) Date of publication of application: **05.07.2023**
(21) Application number: 21861248.9
(22) Date of filing: 12.08.2021
(51) Int. Cl.: A61B 5/24, A61B 5/256, A61B 5/389

(54) **WEARABLE DEVICE AND DETECTION SYSTEM**

(30) Priority: 27.08.2020 JP 2020143862
(71) Applicant: SHIMADZU CORPORATION, Kyoto-shi, Kyoto 604-8511 (JP)
(72) Inventor: TAKEGAWA, Ryo, Kyoto-shi, Kyoto 604-8511 (JP); MURATA, Koichi, Kyoto-shi, Kyoto 604-8511 (JP); FURUTA, Masafumi, Kyoto-shi, Kyoto 604-8511 (JP)
(74) Representative: Kilian Kilian & Partner
(86) International application number: PCT/JP2021/029702
(87) International publication number: WO 2022/044820

(57) **Abstract**

A wearable device (100) wearable by a subject includes: a base (130); a first arm (111) extending from the base; a second arm (112) extending from the base; a first cable (141) disposed along the first arm; a first sensor (101) that is connected to a first end of the first cable (141), attached to a body of a subject who is wearing the wearable device (100), and detects physical body data of the subject; and a signal processor that is connected to a second end of the first cable (141) and processes a detection signal from the first sensor (101). The signal processor is attached to the base (130).

## Description

### TECHNICAL FIELD

The present invention relates to a wearable device and a detection system.

### BACKGROUND ART

NPL 1 discloses a system that detects a myoelectric potential of a face of a subject to estimate emotions of the subject based on the myoelectric potential. The system includes: a stationary processor; a plurality of sensors, and cables connecting the processor to the plurality of sensors.

In this system, the plurality of sensors are attached via pads or the like to the subject's face to detect the myoelectric potential of the subject's face. The sensors each output a myoelectric potential signal indicating the myoelectric potential detected thereby to the processor through the cables. The processor estimates the subject's emotion based on the myoelectric potential signal.

### CITATION LIST

### NON PATENT LITERATURE

NPL 1: https://www.jstage.jst.go.jp/article/jjske/advpub/0/advpub_TJSKE-D-17-00012/_pdf/-char/ja

### SUMMARY OF INVENTION

### TECHNICAL PROBLEM

The above-mentioned system requires long cables in order to increase the degree of freedom of the position of the subject who is equipped with the sensors. However, the long cables may become tangled or may interfere with other objects with the sensors attached to the subject's body. Thus, the above-described system causes a problem that the operation to attach the sensors to the subject's body becomes complicated.

An object of the present invention is to provide a wearable device that is improved in attachability of a sensor to a head and neck portion of a subject.

### SOLUTION TO PROBLEM

A wearable device according to an aspect of the present disclosure is attachable to a head and neck portion of a subject. The wearable device includes a base, a first arm, a second arm, a first cable, a first sensor, and a signal processor. The first arm extends from the base. The second arm extends from the base. The first cable is disposed along the first arm. The first sensor is connected to a first end of the first cable and detects physical body data of the subject with the first sensor attached to the subject who is wearing the wearable device. The signal processor is connected to a second end of the first cable and processes a detection signal from the first sensor. The signal processor is attached to the base.

### ADVANTAGEOUS EFFECTS OF INVENTION

According to the present disclosure, it is possible to provide a wearable device configured to have cables extending along a first arm and thereby improved in attachability to a head and neck portion of a subject.

### BRIEF DESCRIPTION OF DRAWINGS

Fig. 1 is a diagram showing a wearable device.
Fig. 2 is a diagram showing a subject wearing the wearable device, which is viewed obliquely from behind.
Fig. 3 is a front view of the subject wearing the wearable device.
Fig. 4 is another perspective view of the wearable device.
Fig. 5 is a block diagram of a detection system according to a present embodiment.
Fig. 6 is an enlarged view of a left ear hook portion of a first arm.
Fig. 7 is an enlarged view of the left ear hook portion in the state in which cables are respectively disposed in groove portions.
Fig. 8 is a diagram schematically showing groove portions and the like of the first arm.
Fig. 9 is an enlarged view of a right ear hook portion of a second arm.
Fig. 10 is a diagram showing an example of a lug.
Fig. 11 is a diagram showing a configuration example of a detection system according to a second embodiment.
Fig. 12 is a diagram showing an example of a cross section of a tube portion serving as a holding portion according to a third embodiment.

### DESCRIPTION OF EMBODIMENTS

The following describes embodiments of the present disclosure in detail with reference to the accompanying drawings, in which the same or corresponding portions are denoted by the same reference characters, and the description thereof will not be repeated.

### <First Embodiment>

### [Configuration of Wearable Device]

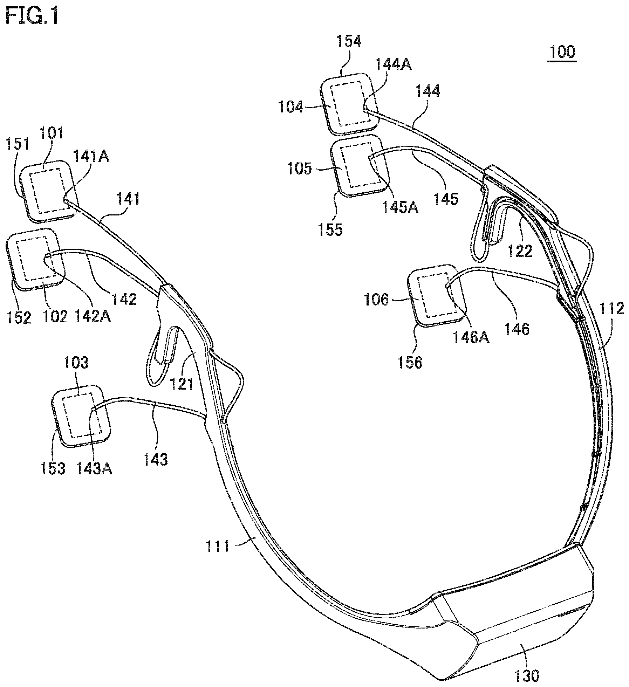

Fig. 1 is a diagram showing a wearable device 100. Referring to Fig. 1, wearable device 100 includes a base 130, a first arm 111, and a second arm 112. First arm 111 and second arm 112 respectively extend from both ends of base 130. Each of first arm 111 and second arm 112 has a curved shape so as to extend along a head of a subject A who is wearing wearable device 100.

First arm 111 and second arm 112 each have a tip end extending to be substantially semicircular in shape. These substantially semicircular arc portions each are hooked over an ear of subject A. The tip end of first arm 111 is provided with a left ear hook portion 121 to be hooked over a left ear of subject. The tip end of second arm 112 is provided with a right ear hook portion 122 to be hooked over a right ear of subject A.

Wearable device 100 further includes a first cable 141, a second cable 142, a third cable 143, a fourth cable 144, a fifth cable 145, and a sixth cable 146. Wearable device 100 also includes a first sensor 101, a second sensor 102, a third sensor 103, a fourth sensor 104, a fifth sensor 105, and a sixth sensor 106, each of which is connected to a corresponding one of first cable 141 to sixth cable 146.

First sensor 101 is incorporated in a first electrode pad 151. Second sensor 102 is incorporated in a second electrode pad 152. Third sensor 103 is incorporated in a third electrode pad 153. Fourth sensor 104 is incorporated in a fourth electrode pad 154. Fifth sensor 105 is incorporated in a fifth electrode pad 155. Sixth sensor 106 is incorporated in a sixth electrode pad 156.

In the state in which wearable device 100 is attached, first sensor 101, second sensor 102, third sensor 103, fourth sensor 104, fifth sensor 105, and sixth sensor 106 are attached to the subject's head with first electrode pad 151, second electrode pad 152, third electrode pad 153, fourth electrode pad 154, fifth electrode pad 155, and sixth electrode pad 156, respectively, interposed therebetween.

First cable 141, second cable 142, and third cable 143 are disposed along first arm 111. As will be described later, first cable 141, second cable 142, and third cable 143 are disposed in groove portions formed in first arm 111. First cable 141 has a first end 141A connected to first sensor 101. Second cable 142 has a first end 142A connected to second sensor 102. Third cable 143 has a first end 143A connected to third sensor 103.

Fourth cable 144, fifth cable 145, and sixth cable 146 are disposed along second arm 112. As will be described later, fourth cable 144, fifth cable 145, and sixth cable 146 are disposed in groove portions formed in second arm 112. Fourth cable 144 has a first end 144A connected to fourth sensor 104. Fifth cable 145 has a first end 145A connected to fifth sensor 105. Sixth cable 146 has a first end 146A connected to sixth sensor 106. Note that the groove portions are provided on surfaces of first arm 111 and second arm 112 that face each other.

In the following description, first sensor 101 to sixth sensor 106 will be collectively referred to as a "sensor group". The sensor group is attached to the head of subject A who is wearing wearable device 100 and detects physical body data of subject A. The physical body data relates to the body of subject A and includes, for example, at least one of a myoelectric potential of the face of the subject, the amount of sweating of the subject, a body temperature of the subject, the amount of moisture in the subject's body, pulse waves of the subject, sounds of the subject, the acceleration of body parts of the subject (subject's mouth or jaw), and brain waves, brain activity, and cardiac potential of the subject. The sounds of the subject include, for example, sounds inside the subject's body (for example, respiratory sound or swallowing sound). The acceleration of the body parts of the subject includes, for example, the acceleration of the subject's muscles.

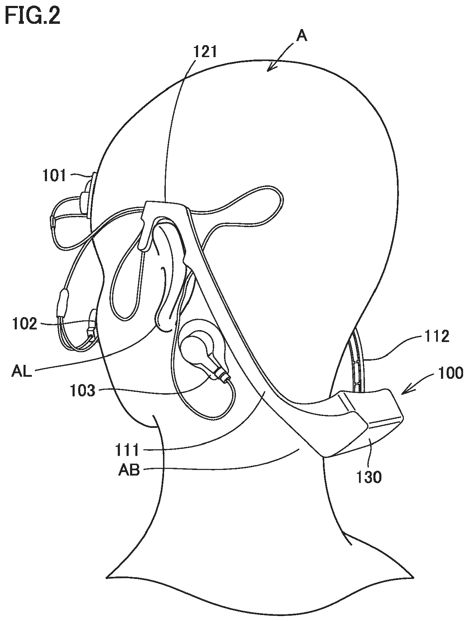

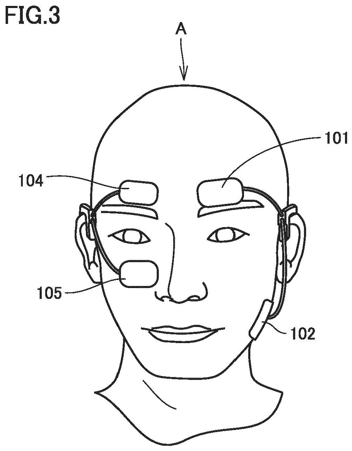

Figs. 2 and 3 each are a diagram showing the state in which wearable device 100 according to the first embodiment is attached to a head and neck portion of subject A and the sensors included in wearable device 100 are attached to subject A. The head and neck portion of the subject is typically a portion above the neck of the subject. Fig. 2 shows subject A viewed obliquely from behind, and Fig. 3 is a front view of subject A. A detection system described later estimates the emotion of subject A who wears wearable device 100. In addition to subject A, an analyzer (particularly not shown) who checks the estimation result of the emotion of subject A also exists. In the following description, the analyzer and subject A will also be collectively referred to as a "user".

In the example in Fig. 2, base 130, first arm 111, second arm 112, first sensor 101, second sensor 102, third sensor 103, and the like are shown. In the attached state, first arm 111 is a left-side arm and second arm 112 is a right-side arm. Further, in the attached state, first arm 111 may be a right-side arm and second arm 112 may be a left-side arm.

Fig. 2 shows an example of the state in which left ear hook portion 121 of first arm 111 is hooked over a left ear AL of subject A. Although not shown in Fig. 2, right ear hook portion 122 (see Fig. 1) of second arm 112 is hooked over the right ear of subject A.

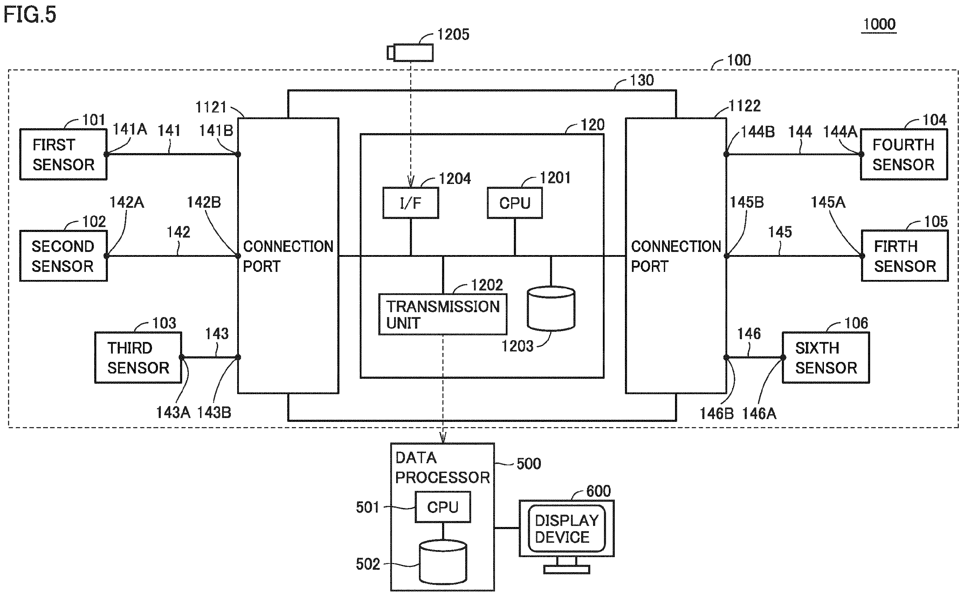

In the state in which wearable device 100 is attached, base 130 is located at a back side head AB (the back side of the head) of subject A. As will be described later, base 130 accommodates a signal processor 120 (see Fig. 5) and thereby has a prescribed weight. Left ear hook portion 121 and right ear hook portion 122 are hooked over the left ear and the right ear, respectively, of subject A. Further, due to the weight of base 130 and the like, subject A can wear wearable device 100 with stability.

Fig. 3 is a front view of subject A who wears wearable device 100. In the example in Fig. 3, first sensor 101, second sensor 102, fourth sensor 104, and fifth sensor 105 are attached to the face of subject A. These sensors are attached, for example, to zygomatic muscles or corrugator muscles to detect the myoelectric potential of the zygomatic muscles or the corrugator muscles.

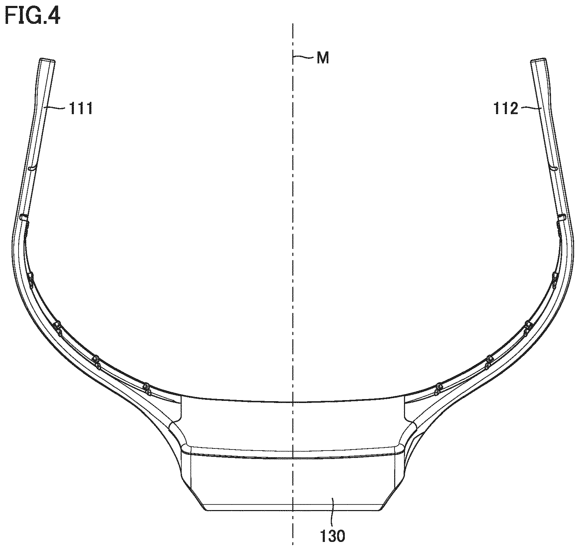

Fig. 4 is a rear view of base 130 of wearable device 100. Fig. 4 fails to show the cables and the sensors. Further, a line bisecting base 130 of wearable device 100 will be hereinafter referred to as a "line M". First arm 111 and second arm 112 are shaped to be in line symmetry with respect to base 130. In other words, first arm 111 and second arm 112 are shaped to have the same length in their extending directions. Thus, subject A can wear wearable device 100 with excellent balance.

### [Detection System]

Fig. 5 is a block diagram of a detection system 1000 including wearable device 100 of the present embodiment. Detection system 1000 of the present embodiment includes wearable device 100, a controller 500, and a display device 600. "Display device 600" of the present embodiment corresponds to an "output device" of the present disclosure.

Signal processor 120 mainly includes a central processing unit (CPU) 1201, a transmission unit 1202, a storage device 1203, and an interface unit 1204. These components are connected to each other through a data bus.

Controller 500 mainly includes a CPU 501 and a storage device 502. Storage devices 1203 and 502 each are formed of a read only memory (ROM), a random access memory (RAM), and the like.

Base 130 includes connection ports 1121 and 1122. Further, signal processor 120 is accommodated in base 130. Among both ends of each of the cables (first cable 141 to sixth cable 146), an end opposite to the first end (first end 141A to first end 146A) to which a corresponding one of the sensors (first sensor 101 to sixth sensor 106) is connected will be hereinafter referred to as a "second end".

A second end 141B of first cable 141, a second end 142B of second cable 142, and a second end 143B of third cable 143 are detachably connected to connection port 1121. A second end 144B of fourth cable 144, a second end 145B of fifth cable 145, and a second end 146B of sixth cable 146 are detachably connected to connection port 1122. Second ends 141B to 146B and connection ports 1121 and 1122 each are configured, for example, by a jack or a connector.

In other words, first cable 141, second cable 142, third cable 143, fourth cable 144, fifth cable 145, and sixth cable 146 are attachable to and detachable from signal processor 120. Note that first cable 141, second cable 142, third cable 143, fourth cable 144, fifth cable 145, and sixth cable 146 may not be attachable to and detachable from signal processor 120, but may be formed integrally with signal processor 120.

First sensor 101, second sensor 102, fourth sensor 104, and fifth sensor 105 are attached to the face of subject A (see Fig. 3) to detect myoelectric potentials at the attached portions. For example, first sensor 101, second sensor 102, fourth sensor 104, and fifth sensor 105 output detection signals indicating the detected myoelectric potentials to signal processor 120.

On the other hand, third sensor 103 and sixth sensor 106 each detect a reference potential used as a reference of the myoelectric potentials. Third sensor 103 and sixth sensor 106 each are attached to a position where the reference potential can be detected. The position where the reference potential can be detected is, for example, behind the ear of subject A. For example, third sensor 103 is attached to a portion behind the left ear of subject A (see Fig. 2), and sixth sensor 106 is attached to a portion behind the right ear of subject A. Potentials detected by third sensor 103 and sixth sensor 106 are used as reference potentials. Third sensor 103 and sixth sensor 106 each output a detection signal indicating the detected reference potential to signal processor 120.

CPU 1201 converts the myoelectric potentials indicated by the detection signals from first sensor 101, second sensor 102, fourth sensor 104, and fifth sensor 105 into potentials based on the reference potentials detected by third sensor 103 and sixth sensor 106. CPU 1201 calculates a difference, for example, between the myoelectric potentials detected by first and second sensors 101 and 102 and the potential (the reference potential) detected by third sensor 103. Further, CPU 1201 calculates a difference, for example, between the myoelectric potentials detected by fourth and fifth sensors 104 and 105 and the potential (the reference potential) detected by sixth sensor 106. CPU 1201 calculates four differential potentials in this way.

Further, CPU 1201 executes a process of changing the information indicating the converted myoelectric potential into a format in which controller 500 can receive the information. CPU 1201 wirelessly transmits the changed signal (control signal) to controller 500 via transmission unit 1202. This control signal includes the four differential potentials. When controller 500 receives the control signal from wearable device 100, CPU 501 executes a prescribed process based on the control signal. The prescribed process is, for example, a process of estimating the emotion of subject A.

CPU 501 of controller 500 extracts four differential potentials from the control signal transmitted from wearable device 100. Further, a table in which four potentials are associated with emotions is generated in advance and stored in storage device 502. CPU 502 refers to the table to extract the emotions corresponding to the four myoelectric potentials indicated by the control signal. Controller 500 outputs the extracted emotion as an estimation result. Note that CPU 501 may estimate the emotion by inputting the myoelectric potentials of four parts of subject A and two reference potentials to the neural network stored in storage device 502. Controller 500 outputs the information indicating the estimation result (estimated emotion) to display device 600. The "estimation result" of the present embodiment corresponds to the "control information" of the present disclosure. Display device 600 displays the information indicating the estimation result (estimated emotion). Controller 500 may output the estimation result to a printer and causes the printer to print the estimation result on a sheet of paper and output the printed sheet of paper. Thereby, the analyzer can recognize the emotion of subject A.

Further, it is also conceivable to adapt a configuration in which wearable device 100 is connected to controller 500 through a cable in a wired manner and transmits a control signal to controller 500 via the cable. In this configuration, however, the cable connecting wearable device 100 to controller 500 may interfere with other objects. Thus, in the present embodiment, wearable device 100 wirelessly transmits a control signal to controller 500. Thereby, occurrence of interference of the cable with other objects can be reduced.

Interface unit 1204 accepts the connection of external storage device 1205 to wearable device 100. In other words, wearable device 100 can be equipped with external storage device 1205. External storage device 1205 is, for example, a universal serial bus (USB) memory. CPU 1201 executes a process of changing the detection signals from first to sixth sensors 101 to 106 into a format in which the detection signals can be stored in external storage device 1205. This process generates detection information. CPU 1201 causes external storage device 1205 to store the detection information (for example, the estimated emotion of subject A). Thus, the analyzer connects external storage device 1205 to another device (for example, a personal computer (PC)) and thereby can recognize the emotion of subject A through this another device.

The system disclosed in the NPL includes a stationary processor, a plurality of sensors, and cables that connect the processor to the plurality of sensors. This system requires long cables in order to increase the degree of freedom of the position of the subject when detecting the physical body data of the subject. However, such long cables may become tangled or may interfere with other objects, and thereby causes a problem that attaching the sensors to the subject's face may become complicated. Further, the system disclosed in the NPL also causes a problem that cables may become tangled or may interfere with other objects also when a subject who is equipped with a plurality of sensors performs a certain operation.

On the other hand, wearable device 100 of the present disclosure is attachable to the head and neck portion of subject A, and thus, wearable device 100 and controller 500 are independent of each other. Wearable device 100 includes sensors (first to sixth sensors 101 to 106) attached to the subject's body, and cables (first to sixth cables 141 to 146) respectively connected to the sensors. Thus, in wearable device 100 of the present disclosure, the length of each cable can be shortened as compared with the above-described system. This consequently can reduce occurrence of entanglement of the cables or interference of the cables with other objects during attachment of the sensors to the head and neck portion of the subject. Thus, according to wearable device 100 of the present disclosure, the attachability of the sensors to the head and neck portion of the subject can be improved. Further, even when a subject equipped with a plurality of sensors performs a certain operation, entanglement of the cables or interference of the cables with other objects can be reduced.

As shown in Fig. 1, wearable device 100 includes cables (for example, first to third cables 141 to 143) disposed along first arm 111, and sensors (for example, first to third sensors 101 to 103) respectively connected to the cables. Also, wearable device 100 further includes cables (for example, fourth to sixth cables 144 to 146) disposed along second arm 112, and sensors (for example, fourth to sixth sensors 104 to 106) respectively connected to the cables. Thus, subject A who wears wearable device 100 can attach the sensors protruding from both sides of the face of subject A to the face of subject A. Therefore, the convenience in attaching the sensors to the face of subject A can be enhanced.

Further, as shown in Fig. 4, first arm 111 and second arm 112 are in line symmetry with respect to base 130 (line M). Thus, subject A can wear wearable device 100 with excellent balance.

As shown in Fig. 1, the tip end of first arm 111 is provided with left ear hook portion 121 shaped to be hooked over the left ear of subject A. The tip end of second arm 112 is provided with right ear hook portion 122 shaped to be hooked over the right ear of subject A. Further, as shown in Fig. 2, in the state in which subject A is wearing wearable device 100, base 130 is located at the head and neck portion of the subject (back side head AB). Thereby, subject A can wear wearable device 100 with stability.

Further, as shown in Fig. 5, each of first cable 141 to sixth cable 146 is attachable to and detachable from signal processor 120. Thus, for example, unnecessary sensors and cables can be detached from wearable device 100, with the result that the convenience in detecting the physical body data of subject A can be enhanced.

In wearable device 100, first sensor 101, second sensor 102, fourth sensor 104, and fifth sensor 105 detect the myoelectric potentials of subject A. Along with this, third sensor 103 and sixth sensor 106 each detect a reference potential of the myoelectric potentials. Thereby, wearable device 100 can calculate the potential (the differential potential) with respect to the reference potential.

### [Groove Portion]

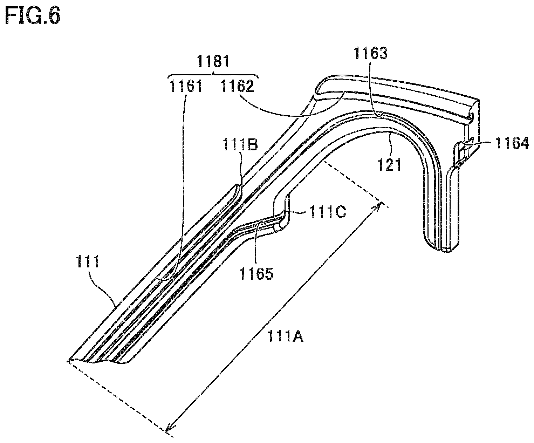

The following describes groove portions formed on the surface of first arm 111. Cables are respectively disposed in these groove portions. Fig. 6 is an enlarged view of left ear hook portion 121 of first arm 111. As shown in Fig. 6, a first groove portion 1161, a second groove portion 1162, a third groove portion 1163, a fourth groove portion 1164, and a fifth groove portion 1165 are formed in first arm 111. In the example in Fig. 6, first groove portion 1161, second groove portion 1162, third groove portion 1163, and fifth groove portion 1165 each are shaped to extend in the extending direction of first arm 111. First arm 111 also includes an extending portion 111A that extends linearly or substantially linearly.

First groove portion 1161 is formed to extend from a portion of first arm 111 that is connected to base 130 to an edge portion 111B of extending portion 111A. Second groove portion 1162 is formed to extend in the extending direction of left ear hook portion 121. There is a relation between first groove portion 1161 and second groove portion 1162 in that they are formed by dividing one groove portion. Third groove portion 1163 is formed across extending portion 111A and left ear hook portion 121. Fourth groove portion 1164 is formed at the distal end of left ear hook portion 121. There is a relation between third groove portion 1163 and fourth groove portion 1164 in that they are formed by dividing one groove portion. Fifth groove portion 1165 is formed to extend from a portion of first arm 111 that is connected to base 130 to an edge portion 111C of extending portion 111A.

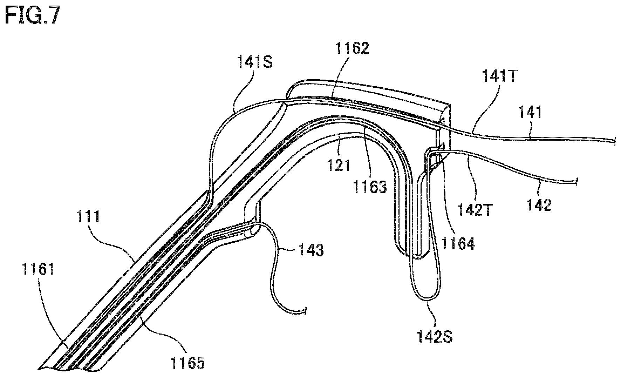

Fig. 7 is an enlarged view of left ear hook portion 121 in the state in which cables are respectively disposed in the groove portions. In the example in Fig. 7, first cable 141 is disposed in first groove portion 1161 and second groove portion 1162. For example, first cable 141 is disposed in first groove portion 1161. Along with this, a portion of first cable 141 that is different from the portion of first cable 141 that is disposed in first groove portion 1161 is disposed in second groove portion 1162 to extend in the extending direction of the first cable. This forms a slack 141S of first cable 141 between first groove portion 1161 and second groove portion 1162. In this way, the positional relation between first groove portion 1161 and second groove portion 1162 allows slack 141S of first cable 141 to be formed between first groove portion 1161 and second groove portion 1162 in the state in which first cable 141 is disposed in first groove portion 1161 and second groove portion 1162. Thus, first cable 141 is disposed in a plurality of groove portions (first groove portion 1161 and second groove portion 1162 in the example in Fig. 7). Also, first sensor 101 is connected to a first end of a protruding portion 141T of first cable 141 that protrudes from first arm 111.

Second cable 142 is disposed in third groove portion 1163 and fourth groove portion 1164. A slack 142S of second cable 142 is formed between third groove portion 1163 and fourth groove portion 1164. In this way, the positional relation between third groove portion 1163 and fourth groove portion 1164 allows slack 142S of second cable 142 to be formed between second groove portion 1162 and fourth groove portion 1164 in the state in which second cable 142 is disposed in third groove portion 1163 and fourth groove portion 1164. Thus, second cable 142 is disposed in a plurality of groove portions (third groove portion 1163 and fourth groove portion 1164 in the example in Fig. 7). Second sensor 102 is connected to a first end of a protruding portion 142T of second cable 142 that protrudes from first arm 111.

Third cable 143 is disposed in fifth groove portion 1165. Unlike first cable 141 and second cable 142, third cable 143 is not disposed in a plurality of groove portions but disposed in one fifth groove portion 1165. Thus, third cable 143 has no slack.

In this way, first cable 141 to third cable 143 are respectively disposed in the groove portions and thereby disposed along first arm 111. Accordingly, first cable 141 to third cable 143 can be disposed along first arm 111 in a relatively simple configuration.

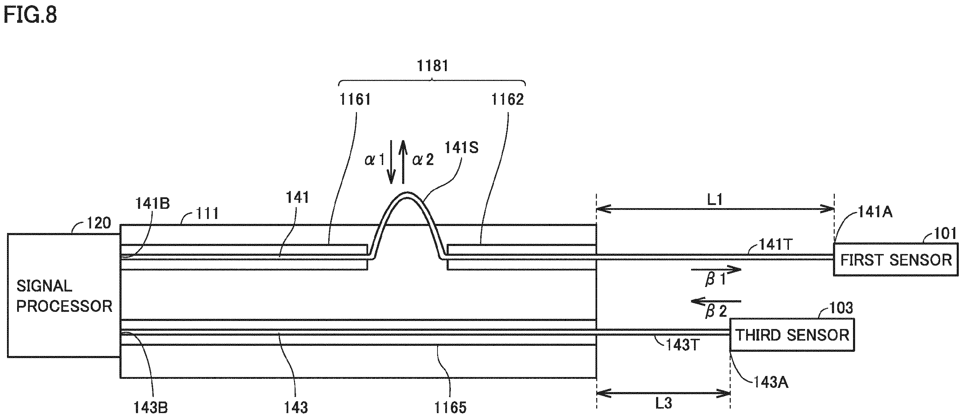

The following describes the function of slack 141S. Fig. 8 schematically shows first groove portion 1161, second groove portion 1162, and a fifth groove portion 1175 of first arm 111. As described with reference to Fig. 7, first cable 141 is disposed in a first groove portion 1171 and a second groove portion 1172, and provided with slack 141S. Third cable 143 is disposed in fifth groove portion 1175. The length of protruding portion 141T of first cable 141 that protrudes from first arm 111 will be hereinafter referred to as a "length L1". The length of a protruding portion 143T of third cable 143 that protrudes from first arm 111 will be hereinafter referred to as a "length L3".

In this case, first cable 141 is formed of an extending conductor and a sheath covering the conductor and extending in the extending direction of the conductor. The sheath is made of a rigid and elastic insulator. Thus, first cable 141 has rigidity and elasticity. Note that the sheath is mainly made of chloroprene rubber, polyvinyl chloride, polyethylene, or the like.

As described above, second end 141B of first cable 141 is connected (fixed) to signal processor 120. Thus, when slack 141S of first cable 141 is pushed by subject A or the like in the direction indicated by an arrow α1, protruding portion 141T of first cable 141 is pushed out in the direction indicated by an arrow β1 since first cable 141 has rigidity and second end 141B of first cable 141 is fixed. In other words, length L1 of protruding portion 141T of first cable 141 increases.

Further, also when protruding portion 141T of first cable 141 is pulled by subject A or the like in the direction indicated by arrow β1, slack 141S moves in the direction indicated by arrow α1 and length L1 of protruding portion 141T of first cable 141 increases.

On the other hand, when slack 141S of first cable 141 is pulled by subject A or the like in the direction indicated by an arrow α2, protruding portion 141T of first cable 141 is pulled in the direction indicated by an arrow β2. In other words, length L1 of protruding portion 141T of first cable 141 decreases. Further, also when protruding portion 141T of first cable 141 is pushed by subject A or the like in the direction indicated by arrow β2, slack 141S moves in the direction indicated by arrow α2 and length L1 of protruding portion 141T of first cable 141 decreases.

In this way, in wearable device 100, a first adjustment portion 1181 capable of adjusting the length of protruding portion 141T of first cable 141 is formed in first arm 111. As shown in Fig. 6, first adjustment portion 1181 is formed of first groove portion 1171 and second groove portion 1172. Length L1 of protruding portion 141T can be adjusted as subject A or the like adjusts slack 141S.

In general, the size of the face of the subject and the muscle positions for facial expression of the subject vary among individuals. In order to allow a sensor to be attached to any subject, it is conceivable to employ a cable having a sufficient length. However, excessively long cables may cause a problem that the cables may become tangled or may collide with other objects. As countermeasures against such a problem, wearable device 100 includes first adjustment portion 1181 capable of adjusting length L1 of protruding portion 141T. Thus, wearable device 100 including first adjustment portion 1181 can reduce occurrence of the above-described problem, and thereby, the user's convenience can be enhanced.

Further, first adjustment portion 1181 is formed of first groove portion 1171 and second groove portion 1172. Therefore, first adjustment portion 1181 can be formed in a relatively simple configuration.

Further, although not particularly shown, an adjustment portion capable of adjusting protruding portion 142T of second cable 142 is also formed in the same configuration as that of first adjustment portion 1181. Specifically, protruding portion 142T of second cable 142 can be adjusted based on slack 142S formed in second cable 142.

The following describes third cable 143 with reference to Fig. 8. As described above, third cable 143 has no slack. As described above, third sensor 103 connected to first end 143A of third cable 143 detects a reference potential. In general, the position where the reference potential can be detected on the subject's face is roughly fixed. Thus, since it is less necessary to change the detection position of the reference potential according to subject, no particular problem occurs even when the length of protruding portion 143T of third cable 143 is fixed. Thus, in the present embodiment, the adjustment portion for adjusting the length of protruding portion 143T of third cable 143 is not provided. In other words, third cable 143 is disposed only in one fifth groove portion 1175. This can eliminates the need to provide an unnecessary adjustment portion (an unnecessary groove portion). Note that the portion of third cable 143 that protrudes from first arm 111 may be able to be adjusted by the extra length of third cable 143 without having to provide an adjustment portion for third cable 143.

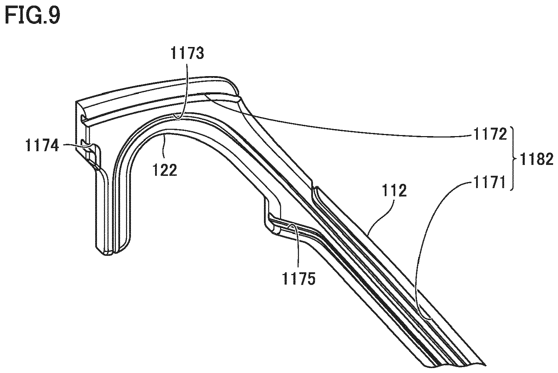

Fig. 9 is an enlarged view of right ear hook portion 122 of second arm 112. As shown in Fig. 9, a first groove portion 1171, a second groove portion 1172, a third groove portion 1173, a fourth groove portion 1174, and a fifth groove portion 1175 are formed in second arm 112. As described with reference to Fig. 4, first arm 111 and second arm 112 are shaped to be in line symmetry with respect to base 130 (line M). Thus, first groove portion 1171 is formed to face first groove portion 1161, second groove portion 1172 is formed to face second groove portion 1162, third groove portion 1173 is formed to face third groove portion 1163, fourth groove portion 1174 is formed to face fourth groove portion 1164, and fifth groove portion 1175 is formed to face fifth groove portion 1165.

Although not particularly shown, fourth cable 144 is disposed in first groove portion 1171 and second groove portion 1172. Further, a slack of fourth cable 144 is formed between first groove portion 1171 and second groove portion 1172. Fifth cable 145 is disposed in third groove portion 1173 and fourth groove portion 1174. Further, a slack of fifth cable 145 is formed between third groove portion 1173 and fourth groove portion 1174. Sixth cable 146 is disposed in fifth groove portion 1175. Sixth cable 146 has no slack.

Therefore, second arm 112 is also provided with: an adjustment portion capable of adjusting the length of the portion of fourth cable 144 that protrudes from second arm 112; and an adjustment portion capable of adjusting the length of the portion of fifth cable 145 that protrudes from second arm 112. For example, second adjustment portion 1182 is formed of first groove portion 1171 and second groove portion 1172. Further, the length of the portion of fourth cable 144 that protrudes from second arm 112 can be adjusted based on the slack of fourth cable 144 that is formed between first groove portion 1171 and second groove portion 1172.

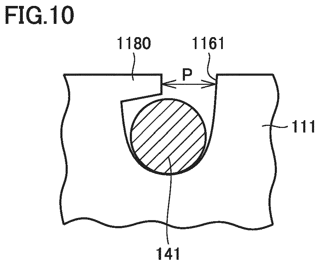

Fig. 10 is a diagram showing an example of cross sections of first groove portion 1161 and first cable 141 disposed in first groove portion 1161. In the example in Fig. 10, a lug 1180 is formed at the edge of the opening of first groove portion 1161. As shown in Fig. 10, lug 1180 is formed in first groove portion 1161 to allow first cable 141 to be retained in first groove portion 1161. As a result, first cable 141 can be prevented from becoming detached from first groove portion 1161. Note that first cable 141 has elasticity as described above. Thus, in the state in which first cable 141 is not disposed in first groove portion 1161, the user pushes first cable 141 through a gap P between lug 1180 and first groove portion 1161 and thereby can place first cable 141 in first groove portion 1161. Further, in the state in which first cable 141 is disposed in first groove portion 1161, the user pulls first cable 141 to be disengaged from gap P and thereby can detach first cable 141 from first groove portion 1161.

One or more lugs 1180 are formed at prescribed positions of first groove portion 1161. Further, one or more lugs 1180 are formed at each of second groove portion 1162 to fifth groove portion 1165, and first groove portion 1171 to fifth groove portion 1175.

Although Fig. 10 illustrates an example in which a lug is provided on one of the left and right sides, but the present invention is not limited thereto, and lugs may be provided alternately on the left and right sides, or lugs may protrude from both the left and right sides at the same position.

### [Second Embodiment]

The first embodiment has been described with regard to the configuration in which first sensor 101 and second sensor 102 detect the same physical body data (myoelectric potential) (see Fig. 5). The second embodiment will be described with regard to a configuration in which the first sensor and the second sensor detect different types of physical body data.

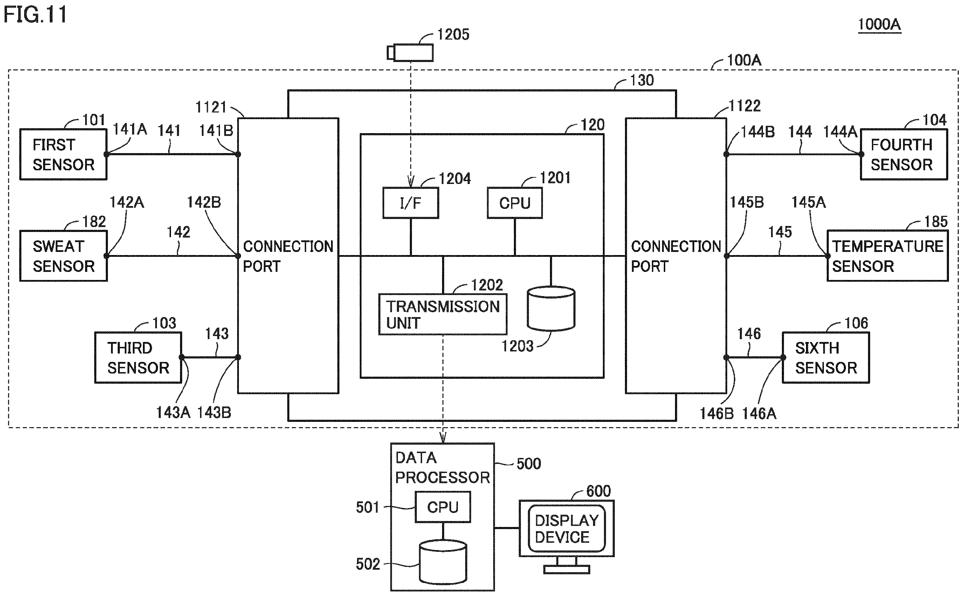

Fig. 11 is a diagram showing a configuration example of a detection system 1000A according to the second embodiment. In comparison between Figs. 5 and 11, second sensor 102 is replaced with a sweat sensor 182 that detects sweating of the subject. Sweat sensor 182 is capable of detecting constituents (for example, lactic acid) contained in human sweat. In comparison between Figs. 5 and 11, fourth sensor 104 is replaced with a temperature sensor 185 that detects the body temperature of the subject. Temperature sensor 185 is formed of a thermistor, for example. Thus, wearable device 100A and detection system 1000A according to the second embodiment allow detection of various physical body data of subject A. Further, the sensors and the cables are attachable to and detachable from wearable device 100, and thereby, a sensor capable of detecting physical body data desired by the user can be attached to wearable device 100.

Further, although the wearable device in the example in Fig. 11 includes first sensor 101 and fourth sensor 104 for detecting the myoelectric potential and third sensor 103 and sixth sensor 106 for detecting the reference potential, the six sensors may detect different types of physical body data.

### [Third Embodiment]

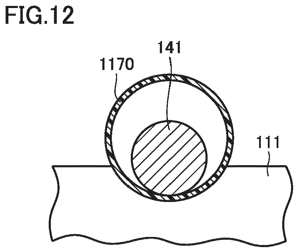

The first embodiment has been described with regard to the configuration in which the holding portion for holding a cable is provided as a groove portion. The third embodiment will be described with regard to a configuration in which the holding portion is provided as a tube portion. The tube portion is formed to be hollow and shaped to extend in the extending direction of the cable. The cable is held inside (in the hollow of) the tube portion. Fig. 12 is a diagram showing an example of a cross section of a tube portion 1170 serving as a holding portion according to the third embodiment. As shown in Fig. 12, tube portion 1170 is formed on the surface of first arm 111 to extend in the extending direction of first cable 141. Further, first cable 141 is disposed to pass through tube portion 1170. Even when the holding portion for holding the cable is tube portion 1170 as in the third embodiment, first cable 141 can be appropriately held in first arm 111.

Further, the first tube portion and the second tube portion may be provided so as to form a slack in first cable 141. Further, a tube portion for holding at least one of second cable 142 to sixth cable 146 may be provided. Further, one cable may be held by a combination of a groove portion and a tube portion. Further, the holding portion for holding the cable may be different in configuration from the groove portion and the tube portion. The holding portion and the cable may be formed of magnets attracting each other, such that the cable may be held by an arm with magnetic force.

Further, instead of holding the cable inside the tube portion, the arm itself may be configured to have a hollow structure through which the cable passes.

### [Modifications]

(1) In the example described in the above embodiments, wearable device 100 is attached to the head of subject A (see Figs. 2 and 3). However, wearable device 100 may have other shapes as long as it is attachable to the body of subject A. For example, wearable device 100 may be shaped to be placed over the neck of subject A.
(2) In the example described with reference to Figs. 6 to 8, the adjustment portion capable of adjusting the length of protruding portion 141T of first cable 141 is formed of a plurality of groove portions. However, the adjustment portion may have other configurations and, for example, may have a winding structure for winding first cable 141.
(3) In the configuration described in the above embodiment, the cable and the sensor are disposed in each of first arm 111 and second arm 112. However, the cable and the sensor may be disposed in one of first arm 111 and second arm 112.

### [Aspects]

It will be understood by those skilled in the art that the above-described exemplary embodiments are illustrative examples of the following aspects.

(Clause 1) A wearable device attachable to a head and neck portion of a subject includes: a base; a first arm extending from the base; a second arm extending from the base; a first cable disposed along the first arm; a first sensor that is connected to a first end of the first cable and detects physical body data of the subject with the first sensor attached to the subject who is wearing the wearable device; and a signal processor that is connected to a second end of the first cable and processes a detection signal from the first sensor. The signal processor is attached to the base.

According the configuration as described above, the wearable device attachable to the head and neck portion of the subject includes sensors to be attached to the subject's body and cables respectively connected to the sensors. Thus, the length of each cable can be shortened, with the result that entanglement of the cables and interference of the cables with other objects can be suppressed. Therefore, a wearable device improved in attachability of the sensors to the head and neck portion of the subject can be provided.

(Clause 2) In the wearable device described in Clause 1, the wearable device further includes a first adjustment portion formed in the first arm and capable of adjusting a length of a portion of the first cable that protrudes from the first arm.

According to the configuration as described above, the length of the portion protruding from the first arm can be adjusted, so that the convenience can be enhanced.

(Clause 3) In the wearable device described in Clause 1 or 2, the wearable device further includes: a second cable disposed along the second arm; and a second sensor that is connected to a first end of the second cable and detects physical body data of the subject with the second sensor attached to a body of the subject who is wearing the wearable device. A second end of the second cable is connected to the signal processor.

According to the configuration as described above, the first sensor and the second sensor can be easily attached to the subject's body.

(Clause 4) In the wearable device described in Clause 3, the wearable device further includes a second adjustment portion formed in the second arm and capable of adjusting a length of a portion of the second cable that protrudes from the second arm.

According to the configuration as described above, the length of the portion protruding from the second arm can be adjusted, so that the convenience can be enhanced.

(Clause 5) In the wearable device described in Clause 2, the first adjustment portion includes: a first holding portion that holds the first cable in an extending direction of the first cable; and a second holding portion that holds a portion of the first cable in the extending direction of the first cable, the portion of the first cable being different from a portion of the first cable that is held by the first holding portion. The length of the portion of the first cable that protrudes from the first arm is adjustable based on a slack of the first cable that is formed between the first holding portion and the second holding portion.

According to the configuration as described above, the first adjustment portion can be formed in a relatively simple configuration.

(Clause 6) In the wearable device described in Clause 5, at least one of the first holding portion and the second holding portion is a groove portion in which the first cable is disposed.

According to the configuration as described above, the first adjustment portion can be formed by the groove portion that can be relatively easily formed.

(Clause 7) In the wearable device described in Clause 6, the groove portion has a lug for retaining the first cable disposed.

According to the configuration as described above, detachment of the first cable from the groove can be reduced.

(Clause 8) In the wearable device described in Clause 5, at least one of the first holding portion and the second holding portion is a tube portion through which the first cable is able to pass.

According to the configuration as described above, the first adjustment portion can be formed by the tube portion that can be relatively easily formed.

(Clause 9) In the wearable device described in Clause 4, the second adjustment portion includes: a third holding portion that holds the second cable in an extending direction of the second cable; and a fourth holding portion that holds, in the extending direction of the second cable, the second cable held by the third holding portion. The length of the portion of the second cable that protrudes from the second arm is adjustable based on a slack of the second cable that is formed between the third holding portion and the fourth holding portion.

According to the configuration as described above, the second adjustment portion can be formed in a relatively simple configuration.

(Clause 10) In the wearable device described in Clause 1 or 2, the first sensor detects a myoelectric potential of the subject, the wearable device further includes: a third cable disposed along the first arm; and a third sensor that is connected to a first end of the third cable and detects a reference potential with the third sensor attached to a body of the subject who is wearing the wearable device. A second end of the third cable is connected to the signal processor.

According to the configuration as described above, the myoelectric potential with reference to the reference potential detected by the third sensor can be detected.

(Clause 11) In the wearable device described in Clause 10, a length of a portion of the third cable that protrudes from the first arm is fixed.

The configuration as described above can eliminate the need to provide the adjustment portion for the third cable.

(Clause 12) In the wearable device described in Clause 3 or 4, the first sensor and the second sensor detect physical body data different in type from each other.

According to the configuration as described above, different types of physical body data can be detected.

(Clause 13) In the wearable device described in any one of Clauses 1 to 12, the first arm and the second arm are shaped to be in line symmetry with respect to the base.

According to the configuration as described above, subject A can wear the wearable device with excellent balance.

(Clause 14) In the wearable device described in any one of Clauses 1 to 13, in the state in which the subject wears the wearable device, a tip end of the first arm is shaped to be hooked over a left ear of the subject, and a tip end of the second arm is shaped to be hooked over a right ear of the subject, and the base is located on a head of the subject.

According to the configuration as described above, physical body data of the head of the subject can be detected.

(Clause 15) In the wearable device described in any one of Clauses 1 to 14, the first cable is attachable to and detachable from the signal processor.

According to the configuration as described above, for example, when the first cable is not required, the first cable can be detached.

(Clause 16) In the wearable device described in any one of Clauses 1 to 15, the signal processor processes a detection signal to generate detection information, an external storage device is attachable to the wearable device, and the wearable device outputs the detection information to the external storage device.

According to the configuration as described above, the detection information generated by the signal processor can be stored in the external storage device.

(Clause 17) In a detection system including: the wearable device described in any one of Clauses 1 to 16; a controller; and a display device, the signal processor processes the detection signal to generate a control signal, and transmits the control signal to the controller, and the controller causes the display device to show information based on the control signal.

According to the configuration as described above, the analyzer or the like can recognize the information based on the control signal.

(Clause 18) In the detection system described in Clause 17, the signal processor wirelessly transmits the control signal to the controller.

For example, when the signal processor transmits the control signal to the controller through wire communication, there occurs a problem, for example, that the cables for such wire communication interfere with other objects. According to the configuration as described above, the signal processor wirelessly transmits the control signal, and thus, the above-described problem can be suppressed.

It should be understood that the embodiments disclosed herein are illustrative and non-restrictive in every respect. The scope of the present disclosure is defined by the terms of the claims, rather than the description in the above embodiments, and is intended to include any modifications within the meaning and scope equivalent to the terms of the claims.

### REFERENCE SIGNS LIST

100 wearable device, 101 first sensor, 102 second sensor, 103 third sensor, 104 fourth sensor, 105 fifth sensor, 106 sixth sensor, 111 first arm, 111A extending portion, 111B, 111C edge portion, 112 second arm, 120 signal processor, 121 left ear hook portion, 122 right ear hook portion, 130 base, 141 first cable, 141S, 142S, slack, 141T, 142T, 143T protruding portion, 142 second cable, 143 third cable, 144 fourth cable, 145 fifth cable, 146 sixth cable, 151 first electrode pad, 152 second electrode pad, 153 third electrode pad, 154 fourth electrode pad, 155 fifth electrode pad, 156 sixth electrode pad, 182 sweat sensor, 185 temperature sensor, 500 controller, 502, 1203 storage device, 600 display device, 1000, 1000A detection system, 1121, 1122 connection port, 1161, 1171 first groove portion, 1162, 1172 second groove portion, 1163, 1173 third groove portion, 1164, 1174 fourth groove portion, 1165, 1175 fifth groove portion, 1170 tube portion, 1180 lug, 1181 first adjustment portion, 1182 second adjustment portion, 1202 transmission unit, 1204 interface unit, 1205 external storage device.

## Claims

1. A wearable device attachable to a head and neck portion of a subject, the wearable device comprising:
a base;
a first arm extending from the base;
a second arm extending from the base;
a first cable disposed along the first arm;
a first sensor that is connected to a first end of the first cable and detects physical body data of the subject with the first sensor attached to the subject who is wearing the wearable device; and
a signal processor that is connected to a second end of the first cable and processes a detection signal from the first sensor, wherein
the signal processor is attached to the base.

2. The wearable device according to claim 1, further comprising a first adjustment portion formed in the first arm and capable of adjusting a length of a portion of the first cable that protrudes from the first arm.

3. The wearable device according to claim 1 or 2, further comprising:
a second cable disposed along the second arm; and
a second sensor that is connected to a first end of the second cable and detects physical body data of the subject with the second sensor attached to a body of the subject who is wearing the wearable device, wherein
a second end of the second cable is connected to the signal processor.

4. The wearable device according to claim 3, further comprising a second adjustment portion formed in the second arm and capable of adjusting a length of a portion of the second cable that protrudes from the second arm.

5. The wearable device according to claim 2, wherein
the first adjustment portion includes
a first holding portion that holds the first cable in an extending direction of the first cable, and
a second holding portion that holds a portion of the first cable in the extending direction of the first cable, the portion of the first cable being different from a portion of the first cable that is held by the first holding portion, and
the length of the portion of the first cable that protrudes from the first arm is adjustable based on a slack of the first cable that is formed between the first holding portion and the second holding portion.

6. The wearable device according to claim 5, wherein at least one of the first holding portion and the second holding portion is a groove portion in which the first cable is disposed.

7. The wearable device according to claim 6, wherein the groove portion has a lug for retaining the first cable disposed.

8. The wearable device according to claim 5, wherein at least one of the first holding portion and the second holding portion is a tube portion through which the first cable is able to pass.

9. The wearable device according to claim 4, wherein
the second adjustment portion includes
a third holding portion that holds the second cable in an extending direction of the second cable, and
a fourth holding portion that holds, in the extending direction of the second cable, the second cable held by the third holding portion, and
the length of the portion of the second cable that protrudes from the second arm is adjustable based on a slack of the second cable that is formed between the third holding portion and the fourth holding portion.

10. The wearable device according to claim 1, wherein
the first sensor detects a myoelectric potential of the subject,
the wearable device further comprises:
a third cable disposed along the first arm; and
a third sensor that is connected to a first end of the third cable and detects a reference potential with the third sensor attached to a body of the subject who is wearing the wearable device, and
a second end of the third cable is connected to the signal processor.

11. The wearable device according to claim 10, wherein a length of a portion of the third cable that protrudes from the first arm is fixed.

12. The wearable device according to claim 3, wherein the first sensor and the second sensor detect physical body data different in type from each other.

13. The wearable device according to claim 1, wherein the first arm and the second arm are shaped to be in line symmetry with respect to the base.

14. The wearable device according to claim 1, wherein
in a state in which the subject wears the wearable device,
a tip end of the first arm is shaped to be hooked over a left ear of the subject, and
a tip end of the second arm is shaped to be hooked over a right ear of the subject, and
the base is located on a head of the subject.

15. The wearable device according to claim 1, wherein the first cable is attachable to and detachable from the signal processor.

16. The wearable device according to claim 1, wherein
the signal processor processes a detection signal to generate detection information,
an external storage device is attachable to the wearable device, and
the wearable device outputs the detection information to the external storage device.

17. A detection system comprising:
the wearable device according to claim 1;
a controller; and
a display device, wherein
the signal processor
processes the detection signal to generate a control signal, and
transmits the control signal to the controller, and
the controller causes the display device to show information based on the control signal.

18. The detection system according to claim 17, wherein the signal processor wirelessly transmits the control signal to the controller.
